# EUROPEAN PATENT SPECIFICATION

(11) **EP 2 352 442 B1**
(45) Date of publication and mention of the grant of the patent: **03.04.2013**
(21) Application number: 08875403.1
(22) Date of filing: 02.12.2008
(51) Int. Cl.: A61B 17/11, A61B 17/115, A61B 1/31, A61B 17/12, A61B 17/00, A61B 17/3205

(54) **A DEVICE FOR HOLLOW ORGAN RESECTION AND CLOSURE**
VORRICHTUNG ZUR RESEKTION UND ZUM VERSCHLIESSEN VON HOHLORGANEN
DISPOSITIF POUR LA RÉSECTION ET LA FERMETURE D'UN ORGANE CREUX

(43) Date of publication of application: 10.08.2011
(73) Proprietor: Ethicon Endo-Surgery, Inc., Cincinnati, Ohio 45242-2839 (US)
(72) Inventor: CSIKY, Laszlo, H-2096 Ueroem (HU); D'ARCANGELO, Michele, I-00142 Roma (IT)
(74) Representative: Leihkauf, Steffen Falk
(86) International application number: PCT/EP2008/066653
(87) International publication number: WO 2010/063313

(56) References cited:
- EP-A- 0 137 685
- EP-A- 1 559 377
- AT-B- 387 513
- DE-C1- 4 133 800
- US-A- 4 476 863
- US-A- 5 314 435
- US-A1- 2004 087 977
- US-A1- 2007 023 475

## Description

The present invention relates to surgical devices and methods for the resection and closure of hollow organs, e.g. bowel, particularly in the treatment of patients with small or medium size carcinoid tumors and in morbid obesity surgery.

Specifically, the present invention relates to surgical devices and methods in the fields of esophageal resection, gastric resection, gastric bypass procedures in morbid obesity surgery, any surgical procedure that requires a so called Roux-en-Y solution, various types of colon-rectal resection and various types of surgical procedures that require a purse-string-suture.

A known surgical technique to perform e.g. the resection of the lower rectum is the so called *low anterior resection* (LAR) which can be performed either by open surgery or laparoscopically using the *Knight-Griffen technique,* also known as double staple technique. Under general anesthesia the rectum is mobilized from the sacrum including division of the lateral ligaments under direct view of the laparoscope or by open surgery. The bowel is divided, e.g. between the sigmoid colon and the rectum using a linear stapler. The lower rectum (containing the tumor) is then transected using a linear or contour stapler.

In this way, a stapled proximal colon stump and a stapled rectal stump are formed, which need to be joined in order to reestablish colon - rectum continuity.

To this end, an anvil of a circular stapler is introduced into the colon stump through a small skin incision, e.g. on the left lower abdomen, and the shaft and staple fastening device of the circular stapler is inserted through the rectal stump via the anus. The shaft and the anvil of the circular stapler are then re-approximated under laparoscopic view, closed and fired. During this end-to-end anastomosis, a circular staple line is fired over the previous lines of staples used to perform the rectal transection (staple crossing) which can lead to leakage at the anastomotic site.

Moreover, in preparation of the anastomosis it is necessary to transport the anvil of the circular stapler to the anastomotic site and deploy it correctly. This would require a rather impossible endoscopic insertion of the anvil from the side opposite the colon stump closure (gastric endoscopy) or deployment of the anvil by laparoscopy or open surgery through an incision of the colon stump proximate to the planned anastomotic site.

Consequently, with general reference to the resection and closure of hollow organs (lumen) and with particular reference to the example of low anterior rectal resection and end-to-end anastomosis of the colon and rectal stumps, there is a need to provide a surgical device and method which overcome the drawbacks of prior art instruments and methods.

Document EP 0 137 685 A2 discloses a surgical device for tubular tissue and organ resection and closure, including an instrument head comprising ann anvil, a staple cartridge and a tissue retention spool located on the rod between the anvil and the cartridge, tissue of the hollow organ is secured to the spool, the spool further includes two flanges, wherein one flange can be used as a cutting guide. Further the use of a two tissue spools, one for one for the lower tubular tissue structure and one for the upper tubular tissue structure is proposed.

The aim of the present invention is therefore to provide a surgical device for hollow organ resection and closure in a combined endoluminal-laparoscopic or endoluminal-open intervention having features which overcome the drawbacks cited with reference to the prior art. Within the scope of the above aim, a particular aim of the present invention is to propose a hollow organ resection and closure device and method which obviates the need to deploy an anvil of a circular stapler after resection of the lumen.

According to an aspect of the invention, the above aim is achieved by a surgical device for lumen resection and closure, including an instrument head and a connector configured to connect the instrument head to an insertion shaft,
wherein the instrument head comprises:
- a first stump closure plug having a first circumferential tie up surface configured such that the mall of the lumen to be resected can be tied up against it from the outside of the lumen,
- a second stump closure plug having a second circumferential tie up surface configured such that the wall of the lumen to be resected can be tied up against it from the outside of the lumen, so that said first and second tie up surfaces define two adjacent tie up planes,
- a latching member detachably connecting said first stump closure plug and said second stump closure plug and forming a cutting block formed between said first and second tie up planes against which a cutting device can cut during resection of the lumen between said two tie up planes,
   wherein at least one of said first and second stump closure plugs further comprises:
- a circular staple forming surface adapted to cooperate with a circular stapler staple fastening device for forming the ends of staples exiting from the staple fastening device,
   so that said stump closure plug is adapted to act as an anvil of said circular stapler.

The device for hollow organ resection and closure enables the surgeon to close the lumen stumps by tying the lumen wall against the stump closure plugs, to stabilize the lumen during tightening and resection and to use the stump closure plug attached to the lumen as an anvil during a subsequent anastomosis by means of a circular stapler.

This enables the surgeon to create a purse string - like lumen stump which is already attached to an anvil, thereby obviating anvil transport and positioning as well as staple crossing during the end-to-end anastomosis.

In accordance with an aspect of the invention, the latching member can be removably connected to one or both of the first and second stump closure plugs or configured to be cut through by the cutting device, to allow removal of the transected portion of the lumen from the lumen stump intended to be reconnected by anastomosis.

In accordance with a yet further aspect of the invention, the device for hollow organ resection and closure comprises an elongate insertion shaft having a proximal handle portion and a distal end portion defining a longitudinal axis, wherein the instrument head is connected to the distal end portion of the insertion shaft by means of a connector or connecting portion which allows tilting of the instrument head with respect to the insertion shaft distal end portion between a rest position of the instrument head in which said tie up planes and cutting plane are approximately parallel with the longitudinal axis and an operational position of the instrument head in which said tie up planes and cutting plane are transversal, preferably perpendicular to the longitudinal axis.

This allows a reduction of the transversal encumbrance of the instrument head during endoluminal insertion of the device and a correct or desired angular positioning of the instrument head at the resection site.

These and other objects and advantages of the present invention shall be made apparent from the accompanying drawings and the description thereof, which illustrate embodiments of the invention and, together with the general description of the invention given above, and the detailed description of the embodiments given below, serve to explain the principles of the present invention.
- Figure 1 is a schematic longitudinal cross-sectional view of a device for hollow organ resection and closure according to an embodiment of the invention, in a rest configuration;
- Figure 2 is a schematic longitudinal cross-sectional view of the device in figure 1, in an operational configuration;

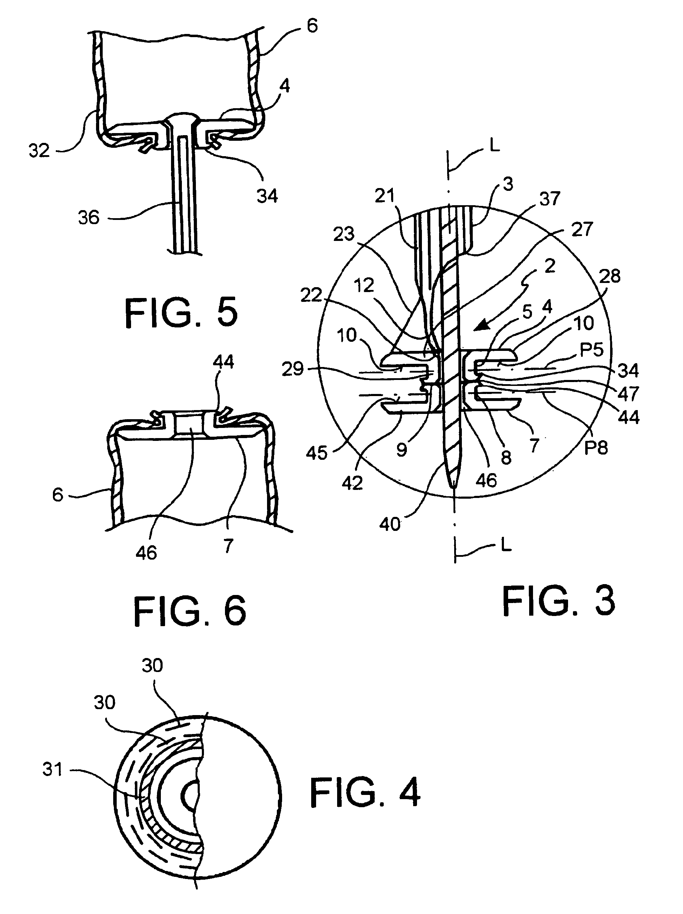

- Figure 3 is an enlarged view of detail III in figure 2;
- Figure 4 is a schematic cross-sectional view of an instrument head of the device in figure 1 and 2 according to sectional plane IV-IV;
- Figure 5 is a schematic cross-sectional view, showing a first lumen stump after resection with attached a first stump closure plug of the device in figure 1;
- Figure 6 is a schematic cross-sectional view, showing a second lumen stump after resection with attached a second stump closure plug of the device in figure 1;
- Figures 7 to 11 illustrate the use of the device in figure 1 for hollow organ resection, closure and anastomosis;
- Figures 14, 15A, 15B illustrate an example of using the device in figure 1 in a gastro-jejunostomy for gastric resection or gastric by-pass;

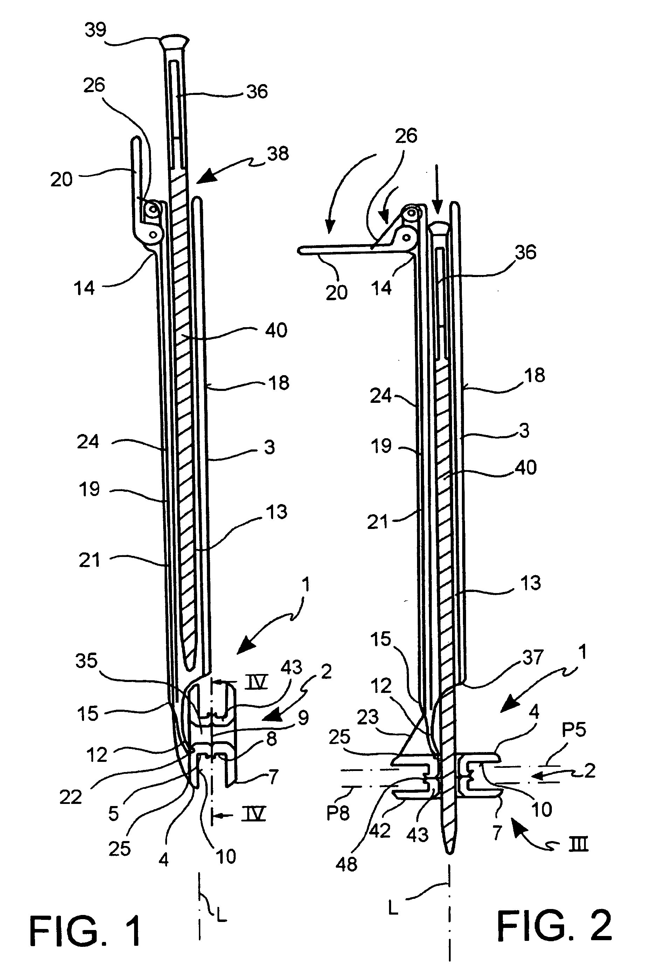

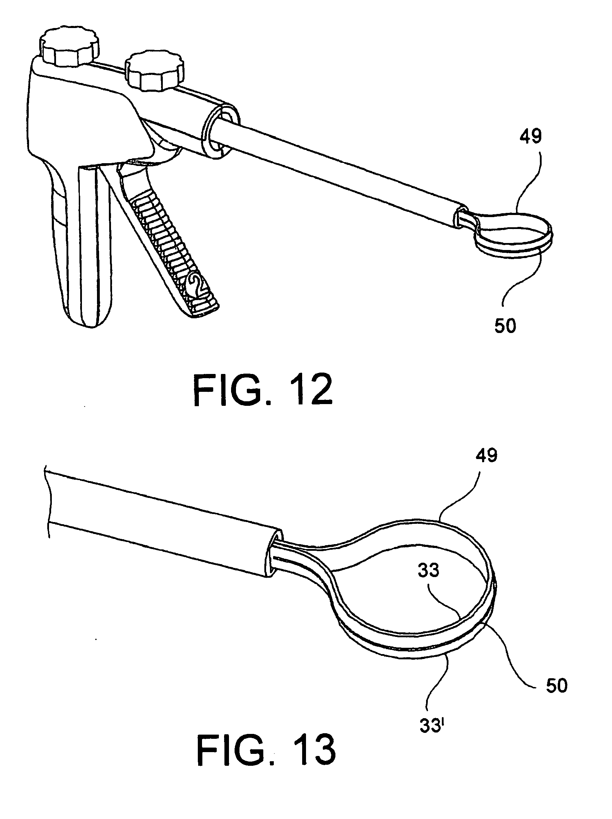

- Figures 12, 13 illustrate a tightening snare and cutting device adapted to be used during lumen resection and closure together with the device in figure 1;

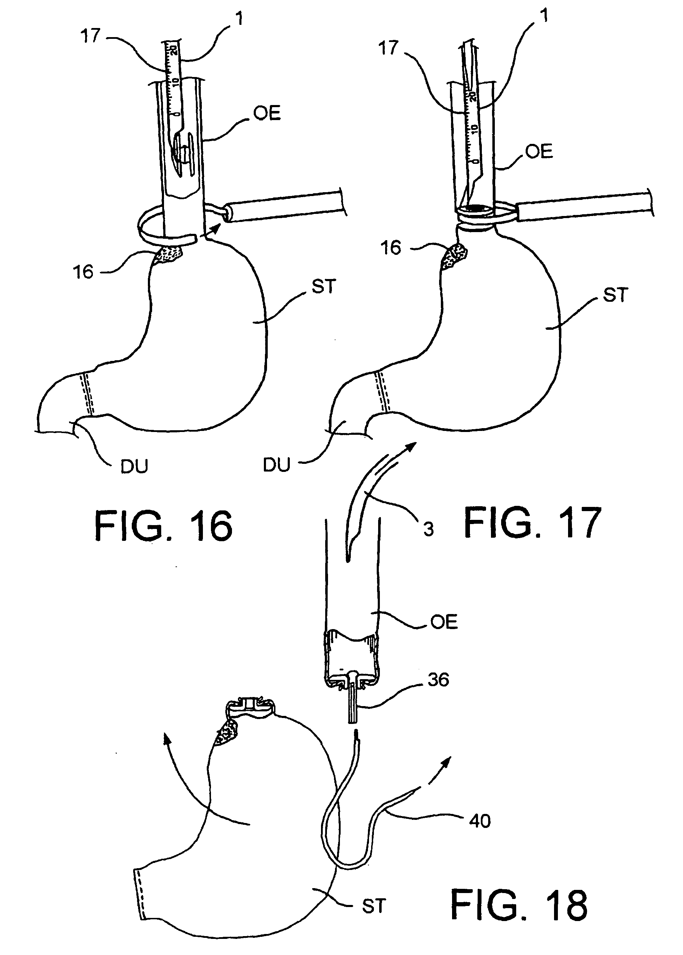

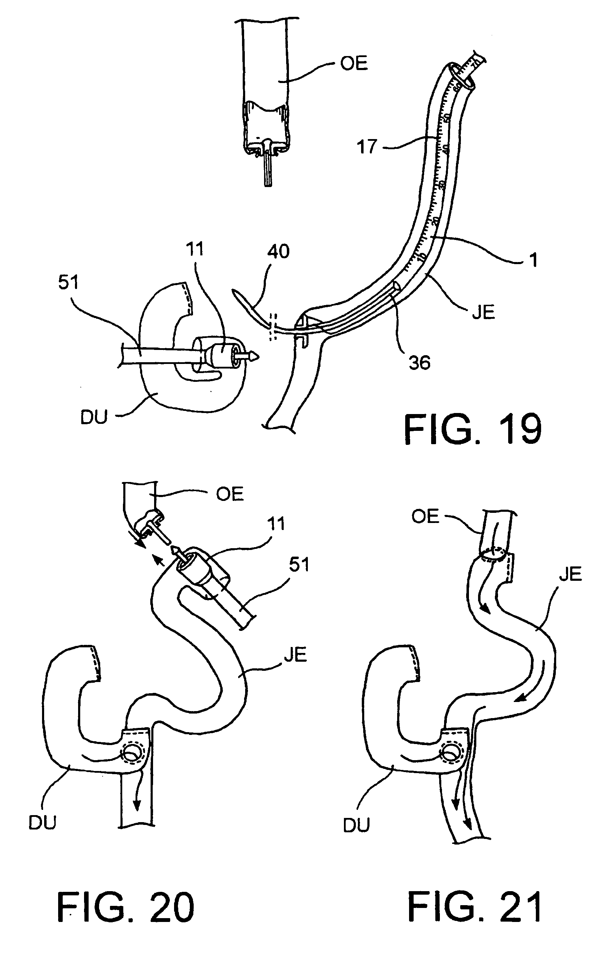

- Figures 16 to 21 illustrate the use of the device for hollow organ resection and closure in an esophagostomy and subsequent esophago-jejunostomy and duodeno-jejunostomy during a total gastrectomy;

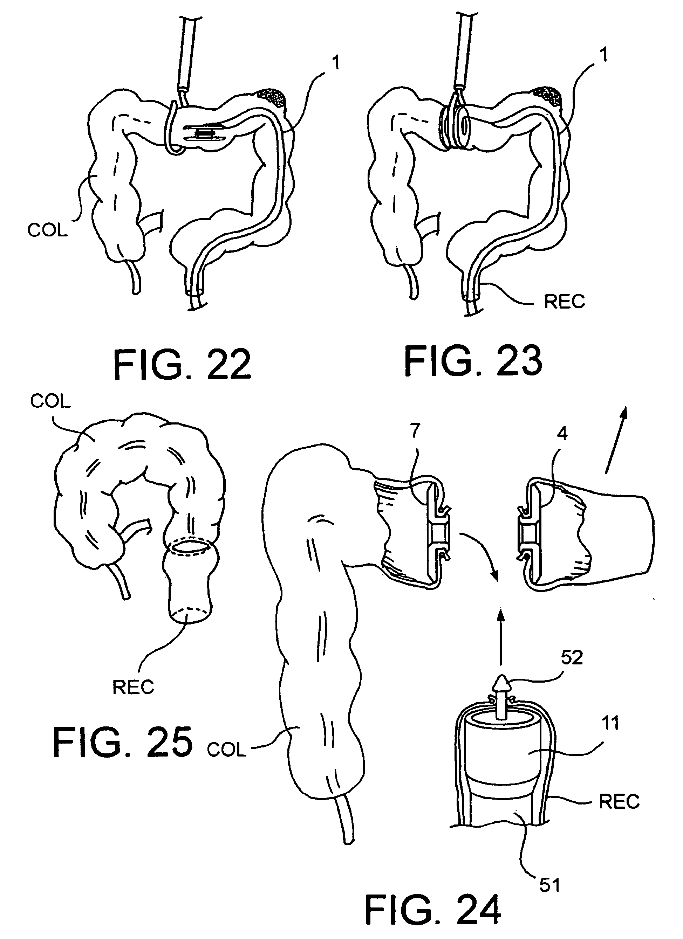

- Figures 22 to 24 illustrate the use of the device for hollow organ resection and closure in a colonostomy and subsequent anastomosis of the healthy stumps of the colon.

Referring to the drawings where like numerals denote like components throughout the several views, figure 1 depicts a surgical device 1 for hollow organ resection and closure.

The device 1 comprises an instrument head 2 connected or connectable to an elongate insertion shaft 3. The instrument head 2 comprises a first stump closure plug 4 with a first circumferential tie up surface 5 configured such that the wall 6 of the lumen to be resected can be tied up against it from the outside of the lumen and a second stump closure plug 7 with a second circumferential tie up surface 8 configured such that the wall of the lumen to be resected can be tied up against it from the outside of the lumen. Both first and second stump closure plugs are arranged in such a manner that the first 5 and second tie up surfaces 8 define two adjacent tie up planes P5, P8. A latching member 9 detachably connects the first stump closure plug 4 and the second stump closure plug 7 between the two adjacent tie up planes P5, P8 and may form a cutting block for a cutting device during resection of the lumen between the two tie up planes P5, P8.

In accordance with an aspect of the invention, at least one of the first 4 and second stump closure plugs 7 further comprises a circular staple forming surface 10 adapted to cooperate with a circular stapler staple fastening device 11 for forming the ends of staples exiting from the staple fastening device 11. In this way, the stump closure plug can act both as a lumen resection and closure device and as an anvil of a circular stapler 12 used during a subsequent end-to-end anastomosis.

Throughout the description of the surgical device, the expressions "distal" and "proximal" are referred to the surgeons point of view. With reference to the gastric intestinal tract, the expression "distal" indicates "toward the mouth" and the expression "proximal" indicates "toward the anus".

The insertion shaft 3, for instance an endoscope or a pushing tube, comprises a longitudinally inextensible rigid or flexible tube extending between a proximal handle portion 14 and a distal end portion 15. The insertion shaft 3 may define an internal working channel 13 for the passage of surgical instruments which will be described further below.

An instrument head connector 12 or one or more connecting seats adapted to receive one or more instrument head connectors are provided at the distal end portion 15 of the insertion shaft 3 in order to connect the instrument head 2 firmly to the distal end portion 15 of the insertion shaft 3.

In accordance with an embodiment, the instrument head connector 12 is integrally formed with the distal end portion 15 of the insertion shaft 3.

In accordance with an alternative embodiment, the distal end portion 15 defines a seat for directly engaging the instrument head 2, e.g. by snap-engagement by screwing or by press-fit.

In accordance with a yet further embodiment, at least part of the instrument head 2 is integrally formed with the insertion shaft 3 and connected to the distal end portion thereof by a connector 12, such as a breaking tie, a breaking rib or a breaking wall having points or lines at which a break is intended to occur in order to separate the instrument head 2 from the insertion shaft 3.

In accordance with an embodiment, the distal end portion 15 of the insertion shaft 3 may further comprise one or more optical scopes or window openings in order to enable visualization of the lumen wall, e.g. of a tumor 16 or other lesion and its margins. This enables the surgeon to precisely place the instrument head 2 with respect to a lesion or to a planned anastomotic site and, hence, to better control the location of resection and lumen stump formation.

Moreover, a graduated sequence of marks 17 indicating e.g. a centimeter-scale may be printed, embossed or otherwise applied to an external surface 18 of the insertion shaft 3 to provide an immediate visual indication of the depth of insertion of the insertion shaft and of the distance of distal end portion 15 from the corresponding body opening, e.g. the anal verge or the mouth of a patient.

In order to facilitate the insertion of the device 1 in the body of a patient and reduce the encumbrance of the instrument head 2 during its endoluminal transportation to the resection site, the instrument head connector 12 may be configured to allow the instrument head to move within certain limits with respect to the insertion shaft distal end portion 15.

In accordance with an embodiment, the instrument head 2 can tilt with respect to the distal end portion 15 between a rest position of the instrument head 2 in which the tie up planes P5, P8 are approximately parallel with a longitudinal axis L of the insertion shaft distal end portion 15 and an operational position of the instrument head 2 in which the tie up planes P5, P8 are transversal, preferably perpendicular to the longitudinal axis L.

This allows a reduction of the encumbrance of the instrument head during endoluminal insertion of the device and a correct desired angular positioning of the instrument head 2 at the resection site.

The tilting position of the instrument head with respect to the insertion shaft can be adjusted by means of a manually operable tilting mechanism 19.

The tilting mechanism 19 comprises a manual actuating member 20, e.g. an actuating lever or knob, arranged at the proximal handle portion 14 of the insertion shaft 2 and an actuation transmission means 21 which connects the manual actuating member 20 operatively with the instrument head 2 so that, in response to an actuating movement of manual actuating member 20, the instrument head 2 moves from the rest position to the operational position and, possibly, vice versa.

In accordance with an embodiment, the instrument head 2 is rotatably connected to the insertion shaft 3 to rotate about a fulcrum 22 and the actuating transmission means 21 comprises a control cable 23 which may be guided inside a cable channel 24 formed in the insertion shaft 3 and extending along almost the entire length of the insertion shaft 3 to protect the control cable from other instruments and to protect the surrounding tissue from the control cable, particularly during an actuating movement of the latter.

The control cable 23 has a proximal end 25 connected to the manual actuating member 20 in a manner that an actuating movement of manual actuating member 20 pulls the control cable proximally and a distal end 26 connected to the instrument head 2 eccentrically with respect to the fulcrum 22 and guided by the insertion shaft 3 (e.g. by cable channel 24) and/or by guide surfaces formed at the instrument head 2 such that, in response of a proximal cable pulling movement, the instrument head 2 tilts from the rest position in the operational position.

In accordance with an embodiment, the device 1 comprises means 27 for securing the operational position of the instrument head, for instance end of stroke abutment surfaces formed both in the instrument head 2 and in the distal end portion 15 of the insertion shaft 3 and adapted to define and maintain the operational position of the instrument head 2 when the control cable pulling force is applied.

Alternatively or additionally, the means 27 for securing the operational position of the instrument head may comprise snap engagement means, locking means or hooking means configured to engage the instrument head 2 when it enters the operational position and to prevent it from rotating back towards the rest position.

The first stump closure plug 4 comprises a generally disk- or plug-shaped ring body with a proximal flange 28 and a tubular tie-up portion 29 projecting from the proximal flange 28 in a distal direction, when the instrument head 2 is held in the operational position. The proximal flange 28 forms a distally facing first staple forming surface 10 adapted to cooperate with a circular stapler staple fastening device 11 for forming the ends of staples exiting from the staple fastening device 11. The first staple forming surface 10 comprises at least one, preferably two annular rows of staple forming pockets 30, wherein the single staple forming pockets of one annular row are preferably circumferentially offset with respect to the single staple forming pockets of an adjacent annular row, thereby ensuring a leak tight staple seam.

The first staple forming surface 10 and the rows of staple forming pockets 30 formed therein are arranged radially outside the tie-up portion 29 so that the portion of lumen wall 6 tied against the tie up portion 29 is situated inside the annular staple line defined by the rows of staple forming pockets 30.

In accordance with an embodiment, the first staple forming surface 10 comprises, at least in the region of the staple forming pockets 30 a mechanically resistant surface layer made of e.g. stainless steel, ceramics or hard plastics to assure a correct deformation of the ends of the staples which are preferably made of titanium or titanium alloy wire.

The proximal flange 28 may further comprise a distally facing cutting block ring 31 configured to cooperate with an annular cutting blade of the circular stapler staple fastening device 11 in order to separate the so called tissue doughnut after circular stapling. Cutting block ring 31 is arranged radially inside the annular rows of staple forming pockets 30 but radially outside the tie-up portion 29, so that the portion of lumen wall 6 tied against the tie up portion 29 is situated also inside the annular cutting line defined by the cutting block ring 31. The cutting block ring 31 can be embodied as a plastic ring insert which can be perceivably cut through, thereby providing a feed-back about the complete and successful separation of the tissue doughnut during the anastomosis.

In accordance with an embodiment, the first staple forming surface 10 together with the rows of staple forming pockets 30 and the cutting block ring 31 is substantially flat and circular. Alternatively, it might be wavy or stepped-wavy both in a plane or out of plane, in order to increase the resulting anastomotic passage. The distally protruding tie up portion 29 forms a first external circumferential tie up surface 5 which extends all around the first stump closure plug 4 and is intended to provide an abutment against which the lumen 6 can be tied up from outside in order to form a closure of the first lumen stump.

In accordance with an embodiment, the first tie up surface 5 forms a circumferential groove to facilitate positioning and tightening of a snare 33 which can be positioned about the lumen 6 or hollow organ and subsequently tightened by open surgery or by laparoscopy. Thanks to the circumferential groove, during tightening the snare 33, the latter is biased to position itself exactly above the first tie up surface 5 and a ring of the lumen 6 is pressed between the snare 33 and the first tie up surface 5 inside the circumferential groove.

The tie up portion 29 of the first stump closure plug 4 may further comprise a circumferentially continuous or interrupted distal limit edge 34 which protrudes radially outside (with respect to a proximal-distal direction of the instrument head) and prevents the tightening snare 33 from slipping off the stump closure plug 4 after lumen resection.

A central passage zone 35 is formed in the first stump closure plug 4 and configured to receive a connecting shaft 36 which might be initially separate from the first stump closure plug 4 and inserted in the central passage zone 35 thereof in order to couple it to the staple fastening assembly 11 of a circular stapler so that the first stump closure plug 4 can act as anvil of the stapler during the end-to-end anastomosis. Such passage zone 35 can be embodied as a channel or, alternatively, as a portion made of an easily penetrable material, such as rubber or expanded polymer. Accordingly, the ring body of the first stump closure plug 4 takes its annular shape not necessarily from the beginning, but when pierced through by the stapler connecting shaft 36.

In accordance with an embodiment, the instrument head 2 and particularly the first stump closure plug 4 is connected to the insertion shaft 3 so that, in the operational position, the central passage zone 35 of the first stump closure plug 4 is substantially aligned with a distal exit aperture 37 of the working channel 13, so that the connecting shaft 36 can be transported through the working channel 13 to the first stump closure plug 4 and, guided by the working channel exit aperture 37, inserted in the central passage zone 35 which forms a distal-pull resisting seat for the connecting shaft 36. To this end the device 1 includes a specifically designed connecting shaft device 38 comprising the above said connecting shaft 38 with a proximal coupling portion 39 adapted to engage the first stump closure plug 4 in a pull proof manner, as well as a flexible insertion wire 40 detachably connected to a distal end of the connecting shaft 36 and adapted to be inserted through the working channel 13 of the insertion shaft 3 and through the central passage zone 35 of the first stump closure plug 4 in order to assist the placement of the connecting shaft 36 and to allow the first stump closure plug 4 attached to the lumen wall to be more comfortably pulled in position for the subsequent anastomosis.

As an example the instrument head 2 of device 1 may comprise only one stump closure plug provided with the above described stapler anvil features. In this case, no latching member would be required for connecting a second stump closure plug to the first one and the lumen could be resected distally from the first tie up plane P5. To this end, the tie up portion 29 of the first and only stump closure plug 4 may include a cutting block section arranged distally (surgeons point of view) from the first tie up surface 5, against which a cutting device, e.g. a RF snare, can cut through the lumen wall. This example has the drawback that, after transection of the hollow organ, only the proximal stump would be closed by the first and only stump closure plug 4, while the distal stump (which usually but not necessarily forms part of the hollow organ portion intended to be removed) would remain open. In order to obtain a closure of both lumen stumps, in accordance with the invention, the instrument head 2 comprises a second stump closure plug 7 arranged distally from the first stump closure plug 4 and adapted to close a second (distal) lumen stump 41.

The second stump closure plug 7 comprises a generally disk- or plug-shaped closed or ring-shaped body with a tubular tie-up portion 43.

The tie up portion 43 forms a second external circumferential tie up surface 8 which extends all around the second stump closure plug 7 and is intended to provide an abutment against which the lumen 6 can be tied up from outside in order to form a closure of the second distal lumen stump 41.

In accordance with an embodiment, the second tie up surface 8 forms a circumferential groove to facilitate positioning and tightening of a snare 33' which can be positioned about the lumen 6 or hollow organ and subsequently tightened by open surgery or by laparoscopy. Thanks to the circumferential groove, during tightening the snare 33', the latter is biased to position itself exactly above the second tie up surface 8 and a ring of the lumen 6 is pressed between the snare 33' and the second tie up surface 8 inside the circumferential groove.

In this context it is to be noted, that the present invention contemplates both separate tightening snares 33, 33' for the first and second stump closure plugs or a single tightening snare or tape having a width such as to cover contemporaneously both tie up surfaces and which can be split or cut into two separate snares by means of the same cutting device used during the transection of the lumen.

The tie up portion 43 of the second stump closure plug 7 may further comprise a circumferentially continuous or interrupted proximal limit edge 44 which protrudes radially outside (with respect to a proximal-distal direction of the instrument head) and prevents the tightening snare 33' from slipping off the second stump closure plug 7 after lumen resection.

The second stump closure plug 7 may further comprise a distal flange 42 from which the tie-up portion 43 projects in a proximal direction (when the instrument head 2 is in the operational position). The distal flange 42 can also form a proximally facing second staple forming surface 45 adapted to cooperate with a circular stapler staple fastening device 11 for forming the ends of staples exiting from the staple fastening device 11. The second staple forming surface 45 comprises at least one, preferably two annular rows of staple forming pockets 30, wherein the single staple forming pockets of one annular row are circumferentially offset with respect to the single staple forming pockets of an adjacent annular row, thereby ensuring a leaktight staple seam.

The second staple forming surface 43 and the rows of staple forming pockets 30 formed therein are arranged radially outside the second tie-up portion 43 so that the portion of lumen wall 6 tied against the tie up portion 43 is situated inside the annular staple line defined by the rows of staple forming pockets 30.

In accordance with an embodiment, the second staple forming surface 45 comprises, at least in the region of the staple forming pockets 30 a mechanically resistant surface layer made of e.g. stainless steel, ceramics or hard plastics to assure a correct deformation of the ends of the staples.

The distal flange 42 may further comprise a proximally facing cutting block ring configured to cooperate with an annular cutting blade of the circular stapler staple fastening device 11 in order to separate the tissue doughnut after circular stapling. The cutting block ring of the second stump closure plug 7 is arranged radially inside the annular rows of staple forming pockets 30 but radially outside the tie-up portion 43, so that the portion of lumen wall 6 tied against the tie up portion 43 is situated also inside the annular cutting line defined by the cutting block ring. Similarly to the cutting block ring of the first stump closure plug 4, also the cutting block ring of the second stump closure plug may be embodied as a plastic ring insert which can be perceivably cut through.

The second staple forming surface 45 together with the rows of staple forming pockets and the cutting block ring can be substantially flat and circular or, alternatively, wavy or stepped-wavy both in a plane or out of plane, in order to increase the resulting anastomotic aperture.

A central passage zone 46 is formed in the second stump closure plug 7 and configured to receive a connecting shaft 36 which might be initially separate from the second stump closure plug 7 and inserted in the central passage zone 46 thereof in order to couple it to the staple fastening assembly 11 of a circular stapler so that the second stump closure plug 7 can act as an anvil of the stapler during the end-to-end anastomosis. Such passage zone 46 can be embodied as a channel or, alternatively, as a portion made of an easily penetrable material, such as rubber or expanded polymer. Accordingly, the body of the second stump closure plug 7 can take an annular shape from the beginning or after being pierced through by the stapler connecting shaft 36.

Also with respect to the second stump closure plug 7, the instrument head 2 is preferably connected to the insertion shaft 3 so that, in the operational position, the central passage zone 46 of the second stump closure plug 7 is substantially aligned with the distal exit aperture 37 of the working channel 13, so that the connecting shaft 36 can be transported through the working channel 13 and the first stump closure plug 4 to the second stump closure plug 4 and, guided by the working channel exit aperture 37, inserted in or coupled to the central passage zone 46 which forms a proximal-pull resisting seat for the connecting shaft 36.

When both stump closure plugs 4, 7 are intended to act as anvils during subsequent anstomoses of the corresponding proximal and distal lumen stumps with other organs or parts of organs, a first connecting shaft can be coupled with the second stump closure plug 7 and, after tying and transection of the lumen and separation of the second stump closure plug 7 from the first stump closure plug 4, a second connecting shaft can be coupled to the remaining first stump closure plug 4. Subsequently, the insertion shaft 3 can be detached (by mechanically uncoupling or controlled breakage) from the first stump closure plug 4 and withdrawn from the patient's body.

The first and second stump closure plugs 4,7 can be connected in different ways. In accordance with an embodiment, the stump closure plugs 4, 7 are coaxial to one another and their tie up portions 29, 43 are facing each other. A latching member 9 can be provided, which detachably connects plugs 4, 7 in order to separate the lumen stumps 32, 41 after tightening the lumen against the respective plugs 4, 7 and transecting it.

Such a detachable connection can be obtained by removable engagement, snap-fit or press fit, of the latching member 9 with plugs 4, 7 or by manufacturing the latching member 9 in a material (rubber, polymer) which can be cut through by the same cutting device which is employed for the hollow organ resection.

To this end, the latching member 9 can be integrally formed with one or both stump closure plugs 4, 7 and configured detachable engage the other plug or to form a cutting block for the cutting device, e.g. a laparoscopic radiofrequency snare.

In accordance with an embodiment, the limit edges 34 and 44 of plugs 4, 7 provide circumferential guide surfaces 47 which define alone or together with the latching member 9 a circumferential cutting groove 48 which invites the cutting device to position itself in a predetermined cutting plane between the first and second tie up planes P5, P8 determined by the corresponding first and second tie up surfaces 5, 8.

Turning again to the tightening snares 33, 33', in accordance with an embodiment, toothed zip ties may be used for tying up the lumen 6 against the stump closure plugs 4, 7. The toothed zip ties provide a ratchet like one way and non return tightening. The tie up snares 33, 33' can be applied laparoscopically or by open surgery. In accordance with an embodiment, an internal surface of the tightening snares or a tightening tape destined to face the first and second tie up surfaces 5, 8 are shaped approximately complementary to the shape of the tie up surfaces 5, 8.

In accordance with an embodiment, the tie up snare and the cutting instrument are integrated in a single open surgery or laparoscopic device including the looped tape 49 having a width covering both stump closure plugs 4,7, as well as a cutting snare 50, e.g. a radiofrequency snare, or mechanical or mechanical vibrating cutting snare which is fixed to the looped tape and extends parallel to the extension of tape 49 along a center line of the latter. In this way the tightened tape 49 assures a correct position of the integrated cutting snare (e.g. RF snare 50) during cutting.

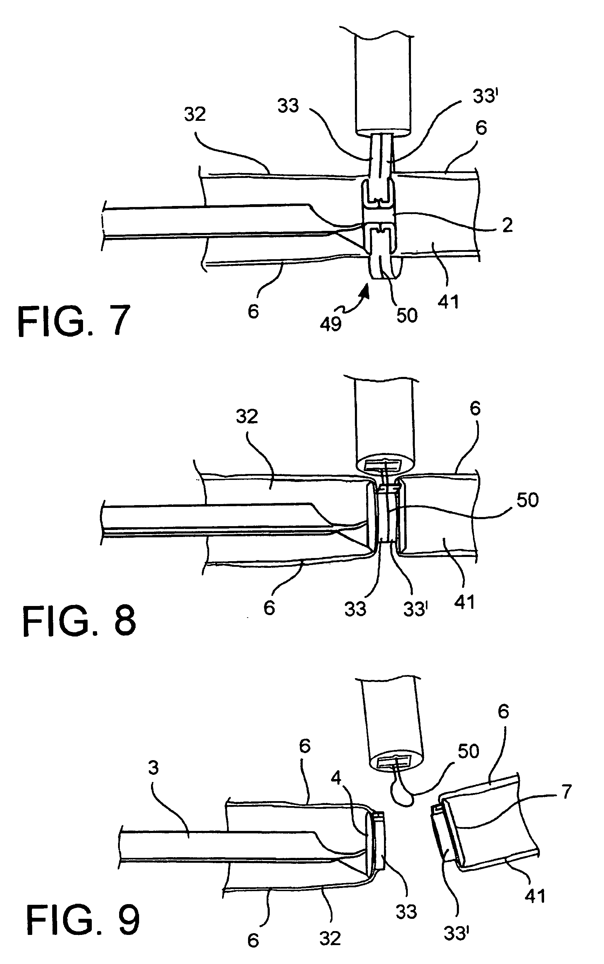

Figures 7 to 9 illustrate the positioning of the surgical device 1, the tightening of the lumen wall 6 against the first and second stump closure plugs and the subsequent transection of the lumen and separation of the stump closure plugs.

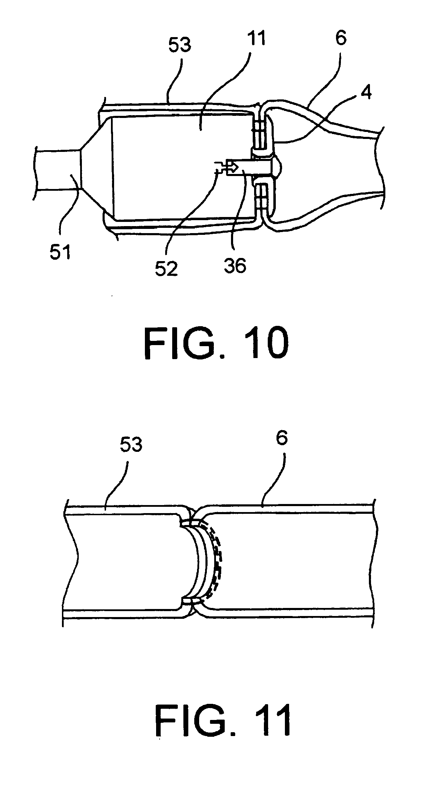

Figures 10 to 11 illustrate the end-to-end anastomosis of at least one of the lumen stumps formed by means of the device 1, using the stump closure plug 4 or 7 attached to the lumen stump as an anvil of a circular stapler 51.

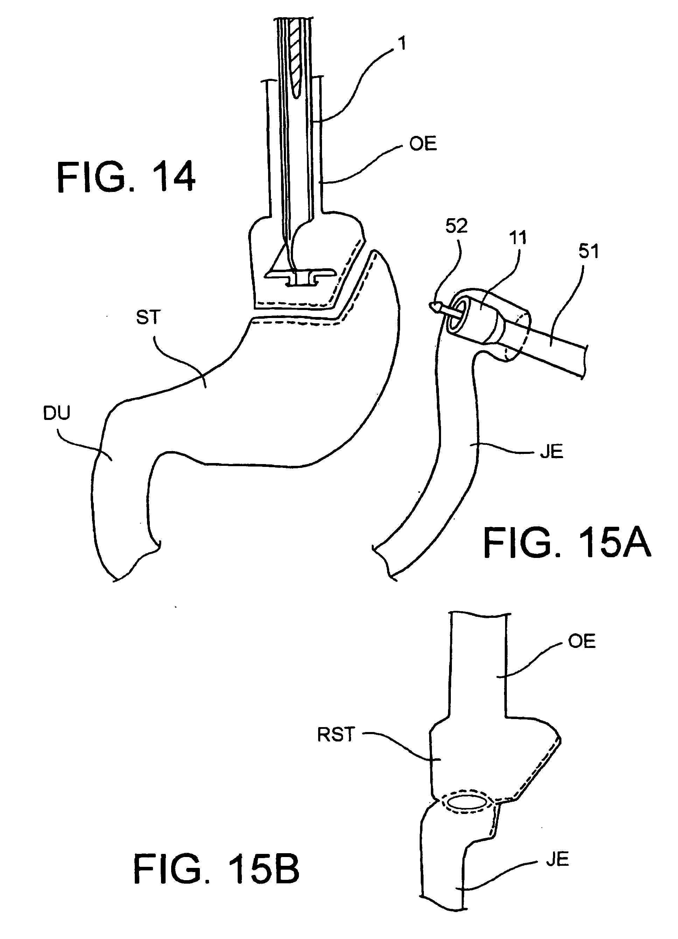

- Figures 14, 15A, 15B illustrate an example of using the device 1 for hollow organ resection and closure in a gastro-jejunostomy for gastric resection or gastric by-pass;
- Figures 22 to 25 illustrate the use of the device 1 for hollow organ resection and closure in a colonostomy and subsequent anastomosis of the healthy stumps of the colon;
- Figures 16 to 21 illustrate an example of using the device 1 for hollow organ resection and closure in an esophagostomy and subsequent esophago-jejunostomy and duodeno-jejunostomy during a total gastrectomy.

In the figures illustrating the above surgical procedures, the following references have been used to indicate anatomical structures and organs:
- OE: esophagus
- ST: stomach
- RST: residual stomach
- DU: duodenum
- JE: jejunum
- COL: colon
- REC: rectum

All illustrated exemplary surgical methods of using the device include the steps of performing a hollow organ or lumen resection, lumen stump closure and lumen stump anastomosis by means of the surgical device 1 according to the invention.

During endoluminal, e.g. transanal, introduction of device 1, the instrument head 2 is tilted in the rest position and the insertion shaft 3 is gently pushed distally (surgeons point of view) until the instrument head 2 reaches the resection site. The insertion depth of device 1 can be visually controlled thanks to the graduated cm scale on the external surface of insertion shaft 3. Then the instrument head is tilted from the rest position into the operational position by manually activating the tilting mechanism 19 of device 1 and held in the operational position. The correct position of the instrument head 2 and particularly of the resection plane thereof can be assisted by direct or indirect endoluminal visualization of the resection site through the optical scopes or window openings of the insertion shaft 3.

After positioning of instrument head 2, first and second tie up snares 33, 33', particularly zip ties, are looped around lumen 6 by laparoscopy or by open surgery (fig. 7) and the tie up snares are tightened about the first and second tie up surfaces 5, 8 of the first stump closure plug 4 and second stump closure plug 7, respectively, thereby cinching the lumen around the plugs 4, 7 which are supported and stabilized by insertion shaft 3 to which they are still firmly connected. In this stage, the circumferentially grooved tie up surfaces of plugs 4, 7 receive the zip ties 33, 33' and assure their correct positioning (fig. 8).

After having the lumen 6 sealed against the plugs 4, 7, resection can be performed between the two tie up planes. To this end, an open surgery or laparoscopic cutting device is brought into position and the rectum is transected between both zip ties 33, 33'. In accordance with an embodiment, the use of an RF snare is contemplated, which is inserted over the lumen 6 and tightened between the first and second plugs 4, 7. Thanks to the guide surfaces 47, a cutting groove 48 is provided between both plugs 4, 7 which enables self alignment of the RF snare during transection.

In accordance with an embodiment of the invention, the cutting instrument cuts completely through both the lumen and the latching member 9, thereby separating the healthy lumen stump from the lumen stump to be removed and the second stump closure plug 7 from the first stump closure plug 4 (figs. 8, 9).

In accordance with another embodiment, the cutting instrument cuts through the lumen 6, but it doesn't transect the latching member 9, which can be subsequently disengage plugs 4, 7 from one another, in order to allow the resulting lumen stumps to be removed from one another. Once the resection is complete (fig. 9, 10), the unhealthy lumen portion containing a lesion is removed from the patient.

The healthy lumen stump can be thoroughly washed and rinsed in order to remove residual tumor cells which might have been accidentally seeded or squeezed into the healthy tissue.

The remaining lumen stump still bound by the tie up snare to the stump closure plug 4 or 7 is now ready for an anastomosis with a further third lumen stump 53 previously prepared by a purse string closure or by linear stapling.

To this end a connecting shaft 36 can be transported through the working channel 13 of the insertion shaft 3 to one or both stump closure plugs 4, 7 of instrument head 2 and connected thereto. After that, the insertion shaft 3 can be detached from the first stump closure plug 4 and removed from the patient's body.

In accordance with an embodiment, the insertion shaft 3, e.g. an anoscope, can be detached from the first stump closure plug 4 by breaking one or more connecting bridges between the anoscope distal end portion 15 and the first stump closure plug 4.

Circular stapling device 51 is now endoluminally introduced until its staple fastening assembly 11 reaches the further lumen stump. A connecting trocar 52 distally projecting from the staple fastening assembly 11 is inserted or pierced through the further lumen stump and connected under laparoscopic view to the connecting shaft 36 of stump closure plug 4, the latter acting as anvil of circular stapling device 51. Then, the stapling device 51 is closed and fired. The cut-out tissue doughnut together with tie up snare 33 is now separated from the lumen joined by anastomosis, but still attached to the stump closure plug.

The circular stapling device 51 together with stump closure plug 4 is then proximally withdrawn from the patient, tissue donuts are checked and a leak test is performed to assure that the anastomosis is air-liquid tight.

The above described device and exemplary method of using the device have many advantages. They obviate the necessity of an additional linear stapler to close the lumen and of additionally inserting of an anvil. The invention makes it further possible to create the lumen stump without purse string suture around the anvil shaft and to reduce transversal encumbrance of the instrument head during endoluminal insertion. Those skilled in the art will appreciate that the device of the present invention is particularly advantageous for the creation of esophago-jejunal and gastro-jejunal anastomosis, as well as for duodeno-jejunal, colo-colic and colo-rectal anastomosis. While the present invention has been illustrated by description of several embodiments and while the illustrative embodiments have been described in considerable detail, it is not the intention to restrict or in any way limit the scope of the appended claims to such detail. The scope of the invention is defined by the appended claims.

## Claims

1. A surgical device (1) for hollow organ resection and closure, including an instrument head (2) wherein the instrument head (2) comprises:
- a first stump closure plug (4) having a first circumferential tie up surface (5) configured such that the wall of a lumen (6) to be resected can be tied up against it from the outside of the lumen (6),
- a second stump closure plug (7) having a second circumferential tie up surface (8) configured such that the wall of the lumen (6) to be resected can be tied up against it from the outside of the lumen (6), so that said first and second tie up surfaces define two adjacent tie up planes (P5, P8),
- a latching member (9) detachably connecting said first stump closure plug (4) with said second stump closure plug (7) and forming
a cutting block between said first and second tie up planes (P5, P8) against which a cutting device can cut during resection of the lumen (6) between said tie up planes (P5, P8),
wherein at least one (4) of said first and second stump closure plugs (4, 7) further comprises:
- a staple forming surface (10) adapted to cooperate with a circular stapler (51) staple fastening device (11) for forming the ends of staples exiting from the staple fastening device (11) so that said stump closure plug (4) can act as an anvil of said circular stapler (51).

2. A surgical device (1) according to claim 1, including an elongate insertion shaft (3) extending between a proximal handle portion (14) and a distal end portion (15), said insertion shaft (3) comprising an instrument head connector (12) provided at the distal end portion (15) for connecting the instrument head (2) to the distal end portion 15 of the insertion shaft 3,
said instrument head connector (12) being configured to allow the instrument head (2) to tilt with respect to the distal end portion (15) between a rest position in which the tie up planes (P5, P8) of the instrument head (2) are approximately parallel with a longitudinal axis (L) of the insertion shaft distal end portion (15) and an operational position in which said tie up planes (P5, P8) are transversal to the longitudinal axis (L).

3. A surgical device (1) according to claim 2, wherein the distal end portion (15) of the insertion shaft (3) comprises an optical scope or window opening adapted to visualize the lumen wall.

4. A surgical device (1) according to claim 2, wherein a graduated sequence of marks (17) indicating a distance-scale is provided on an external surface (18) of the insertion shaft (3).

5. A surgical device (1) according to claim 2, comprising a manually operable tilting mechanism (19) adapted to adjust the tilting position of instrument head (2).

6. A surgical device (1) according to claim 5, wherein said tilting mechanism (19) comprises:
- a manual actuating member (20) arranged at the proximal handle portion (14) of the insertion shaft (2),
- a control cable (23) guided inside a cable channel (24) formed in the insertion shaft (3), said control cable connecting the manual actuating member (20) operatively with the instrument head (2) so that, in response to an actuating movement of manual actuating member (20), the instrument head (2) moves from the rest position to the operational position.

7. A surgical device (1) according to any one of the preceding claims, wherein said first stump closure plug (4) comprises a proximal flange (28) and a tubular tie-up portion (29) projecting from the proximal flange (28) in a distal direction, said tie up portion (29) forming said first external circumferential tie up surface (5) and said proximal flange (28) forming a distally facing first staple forming surface (10) with at least one annular row of staple forming pockets (30) arranged radially outside said tie-up portion (29).

8. A surgical device (1) according to claim 7, wherein said proximal flange (28) further comprises a distally facing cutting block ring (31) configured to cooperate with an annular cutting blade of the circular stapler staple fastening device (11), said cutting block ring (31) being arranged radially inside said annular rows of staple forming pockets (30) but radially outside said tie-up portion (29).

9. A surgical device (1) according to claim 7, in which said first staple forming surface (10) together with the rows of staple forming pockets (30) defines a wavy staple line.

10. A surgical device (1) according to any one of claims 7 to 9, in which the first tie up surface (5) forms a circumferential groove to facilitate positioning and tightening of a tightening snare (33) positioned about the lumen (6).

11. A surgical device (1) according to any one of claims 7 to 10, in which said tie up portion (29) of said first stump closure plug (4) further comprises a radially protruding distal limit edge (34) adapted to prevent the tightening snare (33) from slipping off the stump closure plug (4).

12. A surgical device (1) according to any one of claims 7 to 11, comprising a connecting shaft (36) adapted to couple the first stump closure plug (4) to said staple fastening assembly (11) of a circular stapler so that the first stump closure plug (4) can act as anvil of the stapler.

13. A surgical device (1) according to claim 12, wherein the first stump closure plug (4) is connected to the insertion shaft (3) so that, in the operational position, a central passage zone (35) of the first stump closure plug (4) is substantially aligned with a distal exit aperture (37) of a working channel (13) of insertion shaft (3) and that said connecting shaft (36) can be inserted through the working channel (13) and guided by the working channel exit aperture (37) into the central passage zone (35) which forms a pull-resistant seat for said connecting shaft (36).

14. A surgical device (1) according to any one of the preceding claims, wherein said second stump closure plug (7) comprises a distal flange (42) and a tie-up portion (43) proximally projecting from said distal flange (42), said tie-up portion (43) forming said second external circumferential tie up surface (8) and said distal flange (42) forming a proximally facing second staple forming surface (45) adapted to cooperate with a circular stapler (51) staple fastening device (11) for forming the ends of staples exiting from the staple fastening device (11).

15. A surgical device (1) according to any one of the preceding claims, wherein said latching member (9) is formed from material which can be cut through by the same cutting device which is employed for the hollow organ resection such that said latching member (9) forms said cutting block and can detach said first and second stump closure plugs (4, 7) after resection of the lumen (6).

16. A surgical device (1) according to any one of the preceding claims, wherein said instrument head (2) comprises circumferential guide surfaces (47) which define a circumferential cutting groove (48) adapted to invite the cutting device to position itself in a predetermined cutting plane between the first and second tie up planes (P5, P8).

## Patentansprüche

1. Chirurgisches Gerät (1) zur Hohlorgan-Resektion und Verschließung, umfassend einen Instrumentenkopf (2), wobei der Instrumentenkopf (2) umfasst:
- einen ersten Stumpf-Verschluss-Stopfen (4) mit einer ersten umlaufenden Anbindungsfläche (5), die so konfiguriert ist, dass die Wand von einem zu resektierenden Lumen (6) von der Außenseite des Lumens (6) her dagegen angebunden werden kann,
- einen zweiten Stumpf-Verschluss-Stopfen (7) mit einer zweiten umlaufenden Anbindungsfläche (8), die so konfiguriert ist, dass die Wand des zu resektierenden Lumens (6) von der Außenseite des Lumens (6) her dagegen angebunden werden kann, so dass die erste und die zweite Anbindungsfläche zwei benachbarte Anbindungsebenen (P5, P8) definieren,
- ein Verriegelungselement (9) welches den ersten Stumpf-Verschluss-Stopfen (4) lösbar mit dem zweiten Stumpf-Verschluss-Stopfen (7) verbindet und einen Schneidblock zwischen der ersten und der zweiten Anbindungsebene (P5, P8) bildet, gegen den eine Schneidevorrichtung während der Resektion des Lumens (6) zwischen den Anbindungsebenen (P5, P8) schneiden kann,
wobei wenigstens einer (4) von dem ersten und dem zweiten Stumpf-Verschluss-Stopfen (4, 7) weiter umfasst:
- eine Klammer-Form-Fläche (10), die dazu ausgelegt ist, mit einer Klammer-Befestigungseinrichtung (11) von einem zirkulären Klammergerät (51) zusammen zu wirken, um die Enden von Klammern zu formen, die aus der Klammer-Befestigungseinrichtung (11) austreten, so dass der Stumpf-Verschluss-Stopfen (4) als ein Amboss von dem zirkulären Klammergerät (51) wirken kann.

2. Chirurgisches Gerät (1) nach Anspruch 1, umfassend einen länglichen Einführschaft (3), der sich zwischen einem proximalen Griffabschnitt (14) und einem distalen Endabschnitt (15) erstreckt, wobei der Einführschaft (3) ein Instrumentenkopf-Verbindungselement (12) umfasst, das an dem distalen Endabschnitt (15) vorgesehen ist, um den Instrumentenkopf (2) mit dem distalen Endabschnitt (15) des Einführschafts (3) zu verbinden,
wobei das Instrumentenkopf-Verbindungselement (12) dazu konfiguriert ist, es dem Instrumentenkopf (2) zu ermöglichen, sich in Bezug auf den distalen Endabschnitt (15) zwischen einer Ruheposition, in der die Anbindungsebenen (P5, P8) des Instrumentenkopfs (2) ungefähr parallel zu einer Längsachse (L) von dem distalen Endabschnitt (15) des Einführschafts sind, und einer Betriebsposition zu neigen, in der die Anbindungsebenen (P5, P8) zu der Längsachse (L) transversal sind.

3. Chirurgisches Gerät (1) nach Anspruch 2, wobei der distale Endabschnitt (15) des Einführschafts (3) ein optisches Gerät oder eine Fensteröffnung umfasst, das/die dazu ausgebildet ist, die Lumen-Wand sichtbar zu machen.

4. Chirurgisches Gerät (1) nach Anspruch 2, wobei eine unterteilte Folge von Markierungen (17), welche eine Entfernungsskala angeben, auf einer äußeren Fläche (18) des Einführschafts (3) vorgesehen ist.

5. Chirurgisches Gerät (1) nach Anspruch 2, umfassend einen manuell betreibbaren Neigemechanismus (19), der dazu ausgebildet ist, die Neigungspositionen von dem Instrumentenkopf (2) einzustellen.

6. Chirurgisches Gerät (1) nach Anspruch 5, wobei der Neigemechanismus (19) umfasst:
- ein manuelles Betätigungselement (20), das an dem proximalen Griffabschnitt (14) des Einführschafts (2) angeordnet ist,
- ein Steuerkabel (23), das innerhalb von einem Kabelkanal (24) geführt ist, der in dem Einführschaft (3) ausgebildet ist, wobei das Steuerkabel das manuelle Betätigungselement (20) betriebsmäßig mit dem Instrumentenkopf (2) verbindet, so dass in Reaktion auf eine Betätigungsbewegung des manuellen Betätigungselements (20) der Instrumentenkopf (2) sich von der Ruheposition zu der Betriebsposition bewegt.

7. Chirurgisches Gerät (1) nach einem der vorhergehenden Ansprüche, wobei der erste Stumpf-Verschluss-Stopfen (4) einen proximalen Flansch (28) und einen rohrförmigen Anbindungsabschnitt (29) umfasst, der von dem proximalen Flansch (28) in einer distalen Richtung vorsteht, wobei der Anbindungsabschnitt (29) die erste äußere umlaufende Anbindungsfläche (5) bildet, und der proximale Flansch (28) eine in distale Richtung weisende erste Klammer-Form-Fläche (10) bildet, wobei wenigstens eine ringförmige Reihe von Klammer-Form-Taschen (30) radial außerhalb von dem Anbindungsabschnitt (29) angeordnet ist.

8. Chirurgisches Gerät (1) nach Anspruch 7, wobei der proximale Flansch (28) weiterhin einen in distale Richtung weisenden Schneidblock-Ring (31) umfasst, der dazu konfiguriert ist, mit einer ringförmigen SchneidKlinge der Klammer-Befestigungseinrichtung (11) des zirkulären Klammergeräts zusammen zu wirken, wobei der Schneidblock-Ring (31) radial innerhalb der ringförmigen Reihen von Klammer-Form-Taschen (30) angeordnet ist, aber radial außerhalb von dem Anbindungsabschnitt (29).

9. Chirurgisches Gerät (1) nach Anspruch 7, wobei die erste Klammer-Form-Fläche (10) zusammen mit den Reihen von Klammer-Form-Taschen (30) eine wellenförmige Klammerlinie definiert.

10. Chirurgisches Gerät nach einem der Ansprüche 7 bis 9, wobei die erste Anbindungsfläche (5) eine umlaufende Rille ausbildet, um ein Positionieren und Zuziehen einer um das Lumen (6) positionierten, sich zuziehenden Schlinge (33) zu erleichtern.

11. Chirurgisches Gerät (1) nach einem der Ansprüche 7 bis 10, wobei der Anbindungsabschnitt (29) des ersten Stumpf-Verschluss-Stopfens (4) weiter einen radial hervorstehenden distalen Begrenzungsrand (34) umfasst, der dazu ausgebildet ist, die sich zuziehende Schlinge (33) daran zu hindern, von dem Stumpf-Verschluss-Stopfen (4) herunter zu gleiten.

12. Chirurgisches Gerät (1) nach einem der Ansprüche 7 bis 11, umfassend einen Verbindungsschaft (36), der dazu ausgebildet ist, den ersten Stumpf-Verschluss-Stopfen (4) mit der Klammer-Befestigungsanordnung (11) von einem zirkulären Klammergerät zu koppeln, so dass der erste Stumpf-Verschluss-Stopfen (4) als ein Amboss des Klammergeräts wirken kann.

13. Chirurgisches Gerät (1) nach Anspruch 12, wobei der erste Stumpf-Verschluss-Stopfen (4) mit dem Einführschaft (3) so verbunden ist, dass in der Betriebsposition ein zentraler Durchgangsbereich (35) des ersten Stumpf-Verschluss-Stopfens (4) im Wesentlichen zu einer distalen Austrittsöffnung (37) eines Arbeitskanals (13) des Einführschafts (3) ausgerichtet ist, und dass der Verbindungsschaft (36) durch den Arbeitskanal (13) und, geführt durch die Arbeitskanal-Austrittsöffnung (37), in den zentralen Durchgangsbereich (35) eingesetzt werden kann, der einen zugfesten Sitz für den Verbindungsschaft (36) bildet.

14. Chirurgisches Gerät (1) nach einem der vorhergehenden Ansprüche, wobei der zweite Stumpf-Verschluss-Stopfen (7) einen distalen Flansch (42) und einen Anbindungsabschnitt (43) umfasst, der proximal von dem distalen Flansch (42) vorsteht, wobei der Anbindungsabschnitt (43) die zweite äußere umlaufende Anbindungsfläche (8) bildet und der distale Flansch (42) eine in proximale Richtung weisende zweite Klammer-Form-Fläche (45) bildet, die dazu ausgebildet ist, mit einer Klammer-Befestigungseinrichtung (11) eines zirkulären Klammergeräts (51) zusammen zu wirken, um die Enden von Klammern zu formen, die aus der Klammer-Befestigungseinrichtung (11) austreten.

15. Chirurgisches Gerät (1) nach einem der vorhergehenden Ansprüche, wobei das Verriegelungselement (9) aus einem Material gebildet ist, das durch die gleiche Schneidevorrichtung durchgeschnitten werden kann, die für die Hohlorgan-Resektion verwendet wird, so dass das Verriegelungselement (9) den Schneidblock bildet und nach einer Resektion des Lumens (6) den ersten und den zweiten Stumpf-Verschluss-Stopfen (4, 7) lösen kann.

16. Chirurgisches Gerät (1) nach einem der vorhergehenden Ansprüche, wobei der Instrumentenkopf (2) umlaufende Führungsflächen (47) umfasst, die eine umlaufende Schneid-Rille (48) definieren, die dazu ausgebildet ist, die Schneidevorrichtung dazu einzuladen, sich selbst in einer vordefinierten Schneide-Ebene zwischen der ersten und der zweiten Anbindungsebene (P5, P8) zu positionieren.

## Revendications

1. Dispositif chirurgical (1) pour la résection et la fermeture d'organes creux, incluant une tête d'instrument (2) où la tête d'instrument (2) comprend :
- un premier tampon de fermeture tronqué (4) ayant une première surface de blocage circonférentielle (5) configurée de telle sorte que la paroi d'une lumière (6) à réséquer peut être bloquée contre elle depuis l'extérieur de la lumière (6),
- un second tampon de fermeture tronqué (7) ayant une seconde surface de blocage circonférentielle (8) configurée de telle sorte que la paroi de la lumière (6) à réséquer peut être bloquée contre elle depuis l'extérieur de la lumière (6), de sorte que lesdites première et seconde surfaces de blocage définissent deux plans de blocage adjacents (P5, P8),
- un élément de verrouillage (9) connectant de manière détachable ledit premier tampon de fermeture tronqué (4) avec ledit second tampon de fermeture tronqué (7) et formant un bloc de coupe entre lesdits premier et second plans de blocage (P5, P8) contre lequel un dispositif de coupe peut couper pendant la résection de la lumière (6) entre lesdits plans de blocage (P5, P8),
où au moins l'un (4) desdits premier et second tampons de fermeture tronqués (4, 7) comprend en outre :
- une surface formant agrafes (10) adaptée pour coopérer avec un dispositif de saisie d'agrafes (11) d'agrafeuse circulaire (51) pour former les extrémités d'agrafes sortant du dispositif de saisie d'agrafes (11) de sorte que ledit tampon de fermeture tronqué (4) peut jouer le rôle d'enclume de ladite agrafeuse circulaire (51).

2. Dispositif chirurgical (1) selon la revendication 1, incluant une tige d'insertion allongée (3) s'étendant entre une partie formant poignée proximale (14) et une partie terminale distale (15), ladite tige d'insertion (3) comprenant un connecteur de tête d'instrument (12) disposé au niveau de la partie terminale distale (15) pour connecter la tête d'instrument (2) à la partie terminale distale (15) de la tige d'insertion (3), ledit connecteur de tête d'instrument (12) étant configuré pour permettre à la tête d'instrument (2) de basculer par rapport à la partie terminale distale (15) entre une position de repos dans laquelle les plans de blocage (P5, P8) de la tête d'instrument (2) sont approximativement parallèles à un axe longitudinal (L) de la partie terminale distale (15) de la tige d'insertion et une position opérationnelle dans laquelle lesdits plans de blocage (P5, P8) sont transversaux à l'axe longitudinal (L).

3. Dispositif chirurgical (1) selon la revendication 2, où la partie terminale distale (15) de la tige d'insertion (3) comprend une ouverture formant domaine ou fenêtre optique adaptée pour visualiser la paroi de la lumière.

4. Dispositif chirurgical (1) selon la revendication 2, où une suite graduée de marques (17) indiquant une échelle de distance est disposée sur une surface externe (18) de la tige d'insertion (3).

5. Dispositif chirurgical (1) selon la revendication 2, comprenant un mécanisme de basculement (19) actionnable manuellement adapté pour ajuster la position de basculement de la tête d'instrument (2).

6. Dispositif chirurgical (1) selon la revendication 5, où ledit mécanisme de basculement (19) comprend :
- un élément d'actionnement manuel (20) disposé au niveau de la partie formant poignée proximale (14) de la tige d'insertion (2),
- un câble de commande (23) guidé à l'intérieur d'un canal à câble (24) formé dans la tige d'insertion (3), ledit câble de commande connectant l'élément d'actionnement manuel (20) de manière fonctionnelle avec la tête d'instrument (2) de sorte que, en réponse à un mouvement d'actionnement de l'élément d'actionnement manuel (20), la tête d'instrument (2) se déplace de la position de repos à la position opérationnelle.

7. Dispositif chirurgical (1) selon l'une quelconque des revendications précédentes, où ledit premier tampon de fermeture tronqué (4) comprend une bride proximale (28) et une partie de blocage tubulaire (29) qui fait saillie de la bride proximale (28) dans une direction distale, ladite partie de blocage (29) formant ladite première surface de blocage circonférentielle externe (5) et ladite bride proximale (28) formant une première surface formant agrafes (10) dirigée de manière distale avec au moins une rangée annulaire de poches formant agrafes (30) disposée radialement à l'extérieur de ladite partie de blocage (29).

8. Dispositif chirurgical (1) selon la revendication 7, où ladite bride proximale (28) comprend en outre un anneau de bloc de coupe (31) dirigé de manière distale configuré pour coopérer avec une lame de coupe annulaire du dispositif de saisie d'agrafes (11) d'agrafeuse circulaire, ledit anneau de bloc de coupe (31) étant disposé radialement à l'intérieur desdites rangées annulaires de poches formant agrafes (30) mais radialement à l'extérieur de ladite partie de blocage (29).

9. Dispositif chirurgical (1) selon la revendication 7, où ladite première surface formant agrafes (10) en même temps que les rangées de poches formant agrafes (30) définit une ligne d'agrafes ondulée.

10. Dispositif chirurgical (1) selon l'une quelconque des revendications 7 à 9, où la première surface de blocage (5) forme une rainure circonférentielle pour faciliter le positionnement et le serrage d'un collet de serrage (33) positionné autour de la lumière (6).

11. Dispositif chirurgical (1) selon l'une quelconque des revendications 7 à 10, où ladite partie de blocage (29) dudit premier tampon de fermeture tronqué (4) comprend en outre un bord de limite distale (34) faisant saillie radialement adapté pour empêcher le collet de serrage (33) de glisser du tampon de fermeture tronqué (4).

12. Dispositif chirurgical (1) selon l'une quelconque des revendications 7 à 11, comprenant une tige de connexion (36) adaptée pour coupler le premier tampon de fermeture tronqué (4) audit ensemble de saisie d'agrafes (11) d'une agrafeuse circulaire de sorte que le premier tampon de fermeture tronqué (4) peut jouer le rôle d'enclume de l'agrafeuse.

13. Dispositif chirurgical (1) selon la revendication 12, où le premier tampon de fermeture tronqué (4) est connecté à la tige d'insertion (3) de sorte que, dans la position opérationnelle, une zone de passage centrale (35) du premier tampon de fermeture tronqué (4) est sensiblement alignée avec une ouverture de sortie distale (37) d'un canal de travail (13) de la tige d'insertion (3) et que ladite tige de connexion (36) peut être insérée dans le canal de travail (13) et guidée par l'ouverture de sortie (37) du canal de travail dans la zone de passage centrale (35) qui forme un siège résistant à la traction pour ladite tige de connexion (36).

14. Dispositif chirurgical (1) selon l'une quelconque des revendications précédentes, où ledit second tampon de fermeture tronqué (7) comprend une bride distale (42) et une partie de blocage (43) qui fait saillie de manière proximale depuis ladite bride distale (42), ladite partie de blocage (43) formant ladite seconde surface de blocage circonférentielle externe (8) et ladite bride distale (42) formant une seconde surface formant agrafes (45) dirigée de manière proximale adaptée pour coopérer avec un dispositif de saisie d'agrafes (11) d'agrafeuse circulaire (51) pour former les extrémités d'agrafes sortant du dispositif de saisie d'agrafes (11).

15. Dispositif chirurgical (1) selon l'une quelconque des revendications précédentes, où ledit élément de verrouillage (9) est formé en un matériau qui peut être coupé par le même dispositif de coupe que celui qui est employé pour la résection d'organes creux de telle sorte que ledit élément de verrouillage (9) forme ledit bloc de coupe et peut détacher lesdits premier et second tampons de fermeture tronqués (4, 7) après résection de la lumière (6).

16. Dispositif chirurgical (1) selon l'une quelconque des revendications précédentes, où ladite tête d'instrument (2) comprend des surfaces de guidage circonférentielles (47) qui définissent une rainure de coupe circonférentielle (48) adaptée pour amener le dispositif de coupe à se positionner dans un plan de coupe prédéterminé entre les premier et second plans de blocage (P5, P8).
